(19) Europäisches Patentamt
European Patent Office
Office européen des brevets

(11) EP 3 225 240 A1

(12) **EUROPEAN PATENT APPLICATION**
published in accordance with Art. 153(4) EPC

(43) Date of publication:
**04.10.2017 Bulletin 2017/40**

(21) Application number: **15863656.3**

(22) Date of filing: **27.11.2015**

(51) Int Cl.:
***A61K 31/505*** *(2006.01)* ***A61K 31/47*** *(2006.01)*
***A61K 45/00*** *(2006.01)* ***A61P 3/06*** *(2006.01)*
***A61P 43/00*** *(2006.01)*

(86) International application number:
**PCT/JP2015/083452**

(87) International publication number:
**WO 2016/084949 (02.06.2016 Gazette 2016/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.11.2014 JP 2014240837**

(71) Applicant: **Kowa Company, Ltd.**
**Nagoya-shi, Aichi 460-8625 (JP)**

(72) Inventor: **SHIBATA, Haruki**
**Higashimurayama-shi**
**Tokyo 189-0022 (JP)**

(74) Representative: **Schiener, Jens**
**Wächtershäuser & Hartz**
**Patentanwaltspartnerschaft mbB**
**Weinstraße 8**
**80333 München (DE)**

(54) **MEDICINE**

(57) A medicine for lowering LDL cholesterol is provided. A medicine for lowering LDL cholesterol, the medicine comprising a combination of a statin and (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

EP 3 225 240 A1

## Description

### Technical Field

The present invention relates to a medicine for lowering LDL cholesterol and the like.

### Background Art

In recent years, the onset of dyslipidemia such as hyper-LDL-cholesterolemia, which is considered to belong to so-called lifestyle diseases, tends to increase due to westernized diet. Hyper-LDL-cholesterolemia is regarded as a risk factor for coronary artery diseases (CAD) and cerebrovascular disorder, and the "Guidelines for Prevention of Atherosclerotic Cardiovascular Diseases 2012" of Japan Atherosclerosis Society also illustrate the importance of the management of dyslipidemia.

Dyslipidemia, particularly hyper-LDL-cholesterolemia, has become a class of disease where high satisfactory results for patient have been obtained as a result of development of statins having HMG-CoA reductase inhibitory activity. However, it has been found from the results of numerous large-scale clinical trials that further lowering of the blood LDL cholesterol leads to the prevention of coronary artery diseases (the lower, the better), and thus, more strict lipid control is requested. On the other hand, there are still many patients in whom a blood LDL cholesterol level cannot reach a target level when statins are used alone, and therefore, multidrug therapy is also requested (Non-Patent Literature 1).

Meanwhile, cholesteryl ester transfer protein (CETP) is a glycoprotein having high hydrophobicity and molecular weight of 68, 000 to 74,000, which is produced mainly in liver and small intestine, and this protein has a function of transferring cholesteryl esters in high-density lipoproteins (HDL) to very low-density lipoproteins (VLDL) or low-density lipoproteins (LDL). Therefore, torcetrapib (Patent Literature 1) and anacetrapib (Patent Literature 2), representatives of CETP inhibitors, suppress cholesterol transfer from HDL to LDL and decrease the blood LDL cholesterol level.

The combined administration of statins and CETP inhibitors has already been reported (for example, Patent Literature 3). However, with regard to anacetrapib, a report shows that combined administration of anacetrapib with atorvastatin, which is a kind of statins, led to an enhancement of non-HDL cholesterol lowering effect (Non-Patent Literature 2). By contrast, with regard to combined administration of torcetrapib and atorvastatin, a report shows that an enhancement of the VLDL cholesterol or LDL cholesterol lowering effect was not observed (Non-Patent Literature 3). Thus, the evaluation on the enhancement of the lipid lowering effect obtained from combined administration of statins and CETP inhibitors is undefined.

Meanwhile, it is known that (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid represented by the following Formula (1):

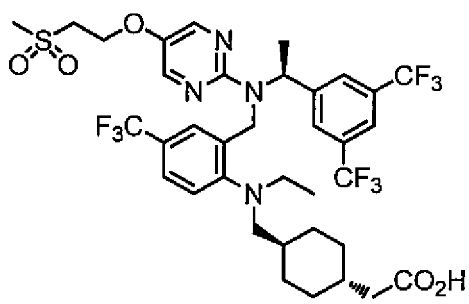

(1)

(hereinafter referred to as pyrimidine compound (1) in the present specification) shows cholesteryl ester transfer protein (CETP) inhibitory effect, and is useful for the prevention or treatment of diseases such as dyslipidemia (Patent Literatures 4 to 6).

However, nothing is specifically known about the combined administration of the pyrimidine compound (1) and statins.

Citation List

Patent Literature

Patent Literature 1: WO 2000/017164 A
Patent Literature 2: WO 2006/014357 A
Patent Literature 3: WO 2004/110406 A
Patent Literature 4: WO 2008/129951 A
Patent Literature 5: WO 2011/152508 A
Patent Literature 6: WO 2012/046681 A

Non Patent Literature

Non-Patent Literature 1: Folia Pharmacol. Jpn., 129, 267-270 (2007)
Non-Patent Literature 2: Kuhnast S., et al., Eur. Heart J., 2014 Aug 20, publication ahead of print
Non-Patent Literature 3: Circulation, 117, 2515-2522 (2008)

Summary of the Invention

Problem to be solved by the Invention

The object of the present invention is to provide a medicine for lowering LDL cholesterol.

Means for Solving the Problem

The present inventor has conducted a thorough investigation in view of such circumstances. As a result, the inventor has found that, when a statin, particularly pitavastatin, a salt thereof, or a solvate of the statin or the salt is used in combination with the pyrimidine compound (1), a salt thereof, or a solvate of the compound or the salt, excellent blood LDL cholesterol lowering effect is provided, thus completed the present invention.

According to the present invention, a medicine for lowering LDL cholesterol is provided. The medicine comprises a combination of a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

Furthermore, according to the present invention, a medicine for lowering LDL cholesterol is provided. The medicine comprises (S)-trans-{4-[({2--[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, and the medicine is administered in combination with a statin for the purpose of lowering LDL cholesterol.

Furthermore, according to the present invention, a medicine for lowering LDL cholesterol is provided. The medicine comprises a statin, and the medicine is administered for the purpose of lowering LDL cholesterol in combination with (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

Effects of the Invention

A combination of a statin and the pyrimidine compound (1), a salt thereof, or a solvate of the compound or the salt, provides excellent blood LDL cholesterol lowering effect and is useful for the prevention and/or treatment of dyslipidemia, particularly hyper-LDL-cholesterolemia.

Brief Description of Drawings

Fig. 1 is a diagram illustrating the X-ray powder diffraction pattern of the crystals of pyrimidine compound (1) hydrochloride obtained in Reference Example 1, steps 1-3.

Fig. 2 is a diagram illustrating the data of thermal analysis measurement (TG-DTA measurement) of the crystals of pyrimidine compound (1) hydrochloride obtained in Reference Example 1, steps 1-3.

Detailed Description of the Invention

The present specification discloses, without limitation, exemplified embodiments of the present inventions as below.

[1] A medicine for lowering LDL cholesterol, the medicine comprising a combination of a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

[2] The medicine according to [1], being in the form of a combination drug comprising both a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

[3] The medicine according to [1] in the form of a kit preparation, comprising a combination of a preparation containing a statin and a preparation containing (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

[4] A medicine for lowering LDL cholesterol comprising (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt,
wherein the medicine is administered in combination with a statin, for the purpose of lowering LDL cholesterol (preferably, for the purpose of preventing and/or treating dyslipidemia; and more preferably, for the purpose of preventing and/or treating hypercholesterolemia).

[5] The medicine according to [4] in the form of a pharmaceutical product for lowering LDL cholesterol comprising the following (A) and (B) :

(A) a medicine for lowering LDL cholesterol containing (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl) phenyl}(ethyl)amino)methyl]cyclohexyl}acetic acid, a salt thereof, or a solvate of the compound or the salt; and
(B) an instruction indicating that the medicine is administered in combination with a statin.

[6] A medicine for lowering LDL cholesterol comprising a statin,
wherein the medicine is administered in combination with (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic, acid, a salt thereof, or a solvate of the compound or the salt, for the purpose of lowering LDL cholesterol (preferably, for the purpose of preventing and/or treating dyslipidemia; and more preferably, for the purpose of preventing and/or treating hypercholesterolemia).

[7] The medicine according to [6] in the form of a pharmaceutical product for lowering LDL cholesterol, comprising the following (A) and (B) :

(A) a medicine for lowering LDL cholesterol containing a statin; and
(B) an instruction indicating that the medicine is administered in combination with (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

[8] The medicine according to any one of [1] to [7], wherein the statin is one or more selected from the group consisting of lovastatin, pravastatin, simvastatin, fluvastatin, atorvastatin, pitavastatin, rosuvastatin, salts thereof, and solvates of the compounds and the salts.

[9] The medicine according to any one of [1] to [7], wherein the statin is atorvastatin, pitavastatin, a salt thereof, or a solvate of the compound or the salt.

[10] The medicine according to any one of [1] to [7], wherein the statin is pitavastatin, a salt thereof, or a solvate of the compound or the salt.

[11] The medicine according to any one of [1] to [10], wherein (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt is one or more member selected from the following <A> to <K>:

<A> hydrochloride of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid;
<B> monohydrochloride of the hydrochloride according to <A>, wherein the hydrochloride is a monohydrochloride;
<C> a crystal of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride;
<D> the crystal according to <C>, wherein the hydrochloride is a monohydrochloride;
<E> the crystal according to <C> or <D>, wherein the X-ray powder diffraction pattern obtainable by irradiation with copper K$\alpha$ radiation shows peaks at one or more diffraction angles (2$\theta$) selected from the group consisting of the vicinity of 14.0 $\pm$ 0.2°, the vicinity of 18.3 $\pm$ 0.2°, the vicinity of 20.1 $\pm$ 0.2°, the vicinity of 20.5 $\pm$ 0.2°, the vicinity of 21.3 $\pm$ 0.2°, the vicinity of 21.8 $\pm$ 0.2°, the vicinity of 23.3 $\pm$ 0.2°, and the vicinity of 24.0 $\pm$ 0.2°;
<F> the crystal according to <C> or <D>, wherein the X-ray powder diffraction pattern obtainable by irradiation with copper K$\alpha$ radiation shows a peak at a diffraction angle (2$\theta$) in the vicinity of 20.5 $\pm$ 0.2°;
<G> the crystal according to <C> or <D>, wherein the X-ray powder diffraction pattern obtainable by irradiation with copper K$\alpha$ radiation shows peaks at diffraction angles (2$\theta$) in the vicinity of 18.3 $\pm$ 0.2° and the vicinity of 20.5 $\pm$ 0.2°;
<H> the crystal according to <C> or <D>, wherein the X-ray power diffraction pattern obtainable by irradiation with copper K$\alpha$ radiation shows peaks at diffraction angles (2$\theta$) in the vicinity of 14.0 $\pm$ 0.2°, the vicinity of 18.3 $\pm$ 0.2°, the vicinity of 20.1 $\pm$ 0.2°, the vicinity of 20.5 $\pm$ 0.2°, the vicinity of 21.3 $\pm$ 0.2°, the vicinity of 21.8 $\pm$ 0.2°, the vicinity of 23.3 $\pm$ 0.2°, and the vicinity of 24.0 $\pm$ 0.2°;
<I> the crystal according to <C> or <D>, wherein the X-ray powder diffraction pattern obtainable by irradiation with copper K$\alpha$ radiation is substantially identical with the pattern illustrated in Fig. 1;
<J> the crystal according to any one of <C> to <I>, showing an endothermic peak in the vicinity of 162 $\pm$ 5.0°C in a differential thermal analysis (DTA); and
<K> the crystal according to any one of <C> to <I>, wherein the results of thermal analysis measurements (differential thermal analysis (DTA) and thermogravimetry (TG)) are substantially identical with the results illustrated in Fig. 2.

[12] A method for preventing and/or treating dyslipidemia (preferably, hypercholesterolemia) comprising a step of administering effective amounts of a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof , or a solvate of the compound or the salt, to a patient of dyslipidemia (preferably, hypercholesterolemia) or a person having a risk of suffering from dyslipidemia (preferably, hypercholesterolemia).
[13] The method according to [12], wherein the statin is one or more selected from the group consisting of lovastatin, pravastatin, simvastatin, fluvastatin, atorvastatin, pitavastatin, rosuvastatin, salts thereof, and solvates of the compounds and the salts.
[14] The method according to [12], wherein the statin is atorvastatin, pitavastatin, a salt thereof, or a solvate of the compound or the salt.
[15] The method according to [12], wherein the statin is pitavastatin, a salt thereof, or a solvate of the compound or the salt.
[16] The method according to any one of [12] to [15], wherein (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromethyl)phenyl} (ethyl) amino)methyl]cyclohexyl}acetic acid, a salt thereof, or a solvate of the compound or the salt is one or more member selected from <A> to <K> described above.
[17] Use of a combination of a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the production of a medicine for lowering LDL cholesterol (preferably, a prophylactic and/or therapeutic agent for dyslipidemia; and more preferably, a prophylactic and/or therapeutic agent for hypercholesterolemia).
[18] Use of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the production of a medicine for lowering LDL cholesterol (preferably, a prophylactic and/or therapeutic agent for dyslipidemia; and more preferably, a prophylactic and/or therapeutic agent for hypercholesterolemia), wherein the medicine is administered in combination with a statin.

[19] Use of a statin for the production of a medicine for lowering LDL cholesterol (preferably, a prophylactic and/or therapeutic agent for dyslipidemia; and more preferably, a prophylactic and/or therapeutic agent for hypercholesterolemia), wherein the medicine is administered in combination with (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

[20] Use according to any one of [17] to [19], wherein the statin is one or more selected from the group consisting of lovastatin, pravastatin, simvastatin, fluvastatin, atorvastatin, pitavastatin, rosuvastatin, salts thereof, and solvates of the compounds and the salts.

[21] Use according to any one of [17] to [19], wherein the statin is atorvastatin, pitavastatin, a salt thereof, or a solvate of the compound or the salt.

[22] Use according to any one of [17] to [19], wherein the statin is pitavastatin, a salt thereof, or a solvate of the compound or the salt.

[23] Use according to any one of [17] to [22], wherein (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt is one or more member selected from <A> to <K> described above.

[24] A combination of a statin and (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for lowering LDL cholesterol (preferably, for the prevention and/or treatment of dyslipidemia; and more preferably, for the prevention and/or treatment of hypercholesterolemia).

[25] (S)-trans-{4-[({2-[({1-[3,5-bis(trifluoromethyl)phenyl]ethyl} {5-[2-(methylsulfonyl)ethoxy]pyrimidin-2-yl}amino)methyl]-4-(triflu oromethyl)phenyl}(ethyl)amino)methyl]cyclohexyl}acetic acid, a salt thereof, or a solvate of the compound or the salt, which is administered in combination with a statin for the purpose of lowering LDL cholesterol (preferably, for the purpose of preventing and/or treating dyslipidemia; and more preferably, for the purpose of preventing and/or treating hypercholesterolemia).

[26] A statin, which is administered in combination with (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the purpose of lowering LDL cholesterol (preferably, for the purpose of preventing and/or treating dyslipidemia; and more preferably, for the purpose of preventing and/or treating hypercholesterolemia).

[27] The compound, a salt thereof, or a solvate of the compound or the salt according to any one of [24] to [26], wherein the statin is one or more member selected from the group consisting of lovastatin, pravastatin, simvastatin, fluvastatin, atorvastatin, pitavastatin, rosuvastatin, salts thereof, and solvates of the compounds and the salts.

[28] The compound, a salt thereof, or a solvate of the compound or the salt according to any one of [24] to [26], wherein the statin is atorvastatin, pitavastatin, a salt thereof, or a solvate of the compound or the salt.

[29] The compound, a salt thereof, or a solvate of the compound or the salt according to any one of [24] to [26], wherein the statin is pitavastatin, a salt thereof, or a solvate of the compound or the salt.

[30] The compound, a salt thereof, or a solvate of the compound or the salt according to any one of [24] to [29], wherein (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt is one or more member selected from <A> to <K> described above.

Regarding the term "statin" according to the present specification, one or more kinds of statins may be used, and specific examples thereof include lovastatin, pravastatin, simvastatin, fluvastatin, atorvastatin, pitavastatin, rosuvastatin, salts thereof, and solvates of the compounds and the salts. One or more kinds of these substances can be used.

The phrase "lovastatin, a salt thereof, or a solvate of the compound or the salt" according to the present specification means to include lovastatin itself, as well as a pharmaceutically acceptable salt of lovastatin (for example, an alkali metal salt such as sodium salt or potassium salt; an alkaline earth metal salt such as calcium salt and magnesium salt; an organic amine salt such as phenethylamine; or an ammonium salt), as well as a solvate of lovastatin or a pharmaceutically acceptable salt thereof with water or an alcohol. One or more kinds of these substances can be used.

Regarding lovastatin, a salt thereof, or a solvate of the compound or the salt, lovastatin (chemical name: [1S-[1α(R*),3α,7β,8β(2S*,4S*),8aβ]]-1,2,3,7,8,8a-hexahydro-3,7-dime thyl-8-[2-(tetrahydro-4-hydroxy-6-oxo-2H-pyran-2-yl)ethyl]-1-naphth alenyl 2-methylbutanoate) is preferred.

Lovastatin, salts thereof, and solvates of the compound and the salts are known compounds, and can be produced by, for example, the method described in US 4231938 B.

The dosage of lovastatin, a salt thereof, or a solvate of the compound or the salt is not particularly limited and can be determined by considering, for example, the gender, age and symptoms of the recipient as appropriate. However,

for example, it is preferable that 1 to 160 mg, more preferably 5 to 120 mg, and particularly preferably 10 to 80 mg, of lovastatin in terms of its free form is taken once or two or more times per day.

The phrase "pravastatin, a salt thereof, or a solvate of the compound or the salt" according to the present specification means to include pravastatin itself, as well as a pharmaceutically acceptable salt of pravastatin (for example, an alkali metal salt such as sodium salt or potassium salt; an alkaline earth metal salt such as calcium salt and magnesium salt; an organic amine salt such as phenethylamine; or an ammonium salt), as well as a solvate of pravastatin or a pharmaceutically acceptable salt thereof with water or an alcohol. One or more kinds of these substances can be used.

Regarding pravastatin, a salt thereof, or a solvate of the compound or the salt, pravastatin sodium (chemical name: Monosodium (3R,5R)-3,5-dihydroxy-7-[(1S,2S,6S,8S,8aR)-6-hydroxy-2-methyl-8[-(2 S)-2-methylbutanoyloxy]-1,2,6,7,8,8a-hexahydronaphthalen-1-yl]hepta noate) is preferred.

Pravastatin, salts thereof, and solvates of the compound and the salts are known compounds, and can be produced by, for example, the methods described in JP Sho 57-2240 A and US 4346227 B.

The dosage of pravastatin, a salt thereof, or a solvate of the compound or the salt is not particularly limited and can be determined by considering, for example, the gender, age and symptoms of the recipient as appropriate. However, for example, it is preferable that 1 to 160 mg, more preferably 2 to 120 mg, and particularly preferably 5 to 80 mg, of pravastatin in terms of pravastatin sodium is taken once or two or more times per day.

The phrase "simvastatin, a salt thereof, or a solvate of the compound or the salt" according to the present specification means to include simvastatin itself, as well as a pharmaceutically acceptable salt of simvastatin (for example, an alkali metal salt such as sodium salt or potassium salt; an alkaline earth metal salt such as calcium salt and magnesium salt; an organic amine salt such as phenethylamine; or an ammonium salt), as well as a solvate of simvastatin or a pharmaceutically acceptable salt thereof with water or an alcohol. One or more kinds of these substances can be used.

Regarding simvastatin, a salt thereof, or a solvate of the compound or the salt, simvastatin (chemical name: (1S,3R,7S,8S,8aR)-8-[2-[(2R,4R)-4-hydroxy-6-oxotetrahydro-2H-pyran-2-yl]ethyl]-3,7-dimethyl-1,2,3,7,8,8a-hexahydronaphthalen-1-yl 2,2-dimethylbutanoate) is preferred.

Simvastatin, salts thereof, and solvates of the compound and the salts are known compounds, and can be produced by, for example, the method described in US 4444784 B.

The dosage of simvastatin, a salt thereof, or a solvate of the compound or the salt is not particularly limited and can be determined by considering, for example, the gender, age and symptoms of the recipient as appropriate. However, for example, it is preferable that 1 to 160 mg, more preferably 2 to 120 mg, and particularly preferably 5 to 80 mg, of simvastatin in terms of its free form is taken once or two or more times per day.

The phrase "fluvastatin, a salt thereof, or a solvate of the compound or the salt" according to the present specification means to include fluvastatin itself, as well as a pharmaceutically acceptable salt of fluvastatin (for example, an alkali metal salt such as sodium salt or potassium salt; an alkaline earth metal salt such as calcium salt and magnesium salt; an organic amine salt such as phenethylamine; or an ammonium salt), as well as a solvate of fluvastatin or a pharmaceutically acceptable salt thereof with water or an alcohol. One kind or a combination of two or more kinds of these substances can be used.

Regarding fluvastatin, a salt thereof, or a solvate of the compound or the salt, fluvastatin sodium (chemical name: (±)-(3RS,5SR,6E)-sodium-7-[3-(4-fluorophenyl)-1-(1-methylethyl)-1H-indol-2-yl]-3,5-dihydroxy-6-heptenoate) is preferred.

Fluvastatin, salts thereof, and solvates of the compound and the salts are known compounds, and can be produced by, for example, the methods described in JP Sho 60-500015 W and US 5354772 B.

The dosage of fluvastatin, a salt thereof, or a solvate of the compound or the salt is not particularly limited and can be determined by considering, for example, the gender, age and symptoms of the recipient as appropriate. However, for example, it is preferable that 1 to 160 mg, more preferably 5 to 120 mg, and particularly preferably 10 to 80 mg, of fluvastatin in terms of its free form is taken once or two or more times per day.

The phrase "atorvastatin, a salt thereof, or a solvate of the compound or the salt" according to the present specification means to include atorvastatin itself, as well as a pharmaceutically acceptable salt of atorvastatin (for example, an alkali metal salt such as sodium salt or potassium salt; an alkaline earth metal salt such as calcium salt and magnesium salt; an organic amine salt such as phenethylamine; or an ammonium salt), as well as a solvate of atorvastatin or a pharmaceutically acceptable salt thereof with water or an alcohol. One kind or a combination of two or more kinds of these substances can be used.

Regarding atorvastatin, a salt thereof, or a solvate of the compound or the salt, atorvastatin calcium hydrate (chemical name: (-)-Monocalcium bis[(3R,5R)-7-[2-(4-fluorophenyl-5-isopropyl-3-phenyl-4-phenylcarba moyl-1H-pyrrol-1-yl)-3,5-dihydroxyheptanoate] trihydrate}) is preferred.

Atorvastatin, salts thereof, and solvates of the compound and the salts are known compounds, and can be produced by, for example, the methods described in JP Hei 3-58967 A and US 5273995 B.

The dosage of atorvastatin, a salt thereof, or a solvate of the compound or the salt is not particularly limited and can be determined by considering, for example, the gender, age and symptoms of the recipient as appropriate.

However, for example, it is preferable that 1 to 160 mg, more preferably 2 to 120 mg, and particularly preferably 5 to 80 mg, of atorvastatin in terms of its free form is taken once or two or more times per day.

The phrase "pitavastatin, a salt thereof, or a solvate of the compound or the salt" according to the present specification means to include pitavastatin itself, as well as a pharmaceutically acceptable salt of pitavastatin (for example, an alkali metal salt such as sodium salt or potassium salt; an alkaline earth metal salt such as calcium salt and magnesium salt; an organic amine salt such as phenethylamine; or an ammonium salt), as well as a solvate of pitavastatin or a pharmaceutically acceptable salt thereof with water or an alcohol. One kind or a combination of two or more kinds of these substances can be used.

Regarding pitavastatin, a salt thereof, or a solvate of the compound or the salt, calcium salt of pitavastatin or a hydrate thereof is preferred, and pitavastatin calcium (chemical name: (+) -monocalcium bis[(3R,5S,6E)-7-[2-cyclopropyl-4-(4-fluorophenyl)-3-quinolyl]-3,5-dihydroxy-6-heptenoate}) or a hydrate thereof (particularly, pentahydrate) is particularly preferred.

Pitavastatin, salts thereof, and solvates of the compound and the salts are known compounds, and can be produced by, for example, the methods described in JP Hei 1-279866 A and US 5856336 B.

The dosage of pitavastatin, a salt thereof, or a solvate of the compound or the salt is not particularly limited and can be determined by considering, for example, the gender, age and symptoms of the recipient as appropriate. However, for example, it is preferable that 0.1 to 16 mg, more preferably 0.5 to 8 mg, and particularly preferably 1 to 4 mg, of pitavastatin in terms of pitavastatin calcium is taken once or two or more times per day.

The phrase "rosuvastatin, a salt thereof, or a solvate of the compound or the salt" according to the present specification means to include rosuvastatin itself, as well as a pharmaceutically acceptable salt of rosuvastatin (for example, an alkali metal salt such as sodium salt or potassium salt; an alkaline earth metal salt such as calcium salt and magnesium salt; an organic amine salt such as phenethylamine; or an ammonium salt), as well as a solvate of rosuvastatin or a pharmaceutically acceptable salt thereof with water or an alcohol. One kind or a combination of two or more kinds of these substances can be used.

Regarding rosuvastatin, a salt thereof, or a solvate of the compound or the salt, rosuvastatin calcium (chemical name: Monocalcium bis((3R,5S,6E)-7-[4-(4-fluorophenyl)-6-isopropyl-2-[methanesulfonyl (methyl)amino]pyrimidin-5-yl]-3,5-dihydroxyhept-6-enoate)) is preferred.

Rosuvastatin, salts thereof, and solvates of the compound and the salts are known compounds, and can be produced by, for example, the methods described in JP Hei 5-178841 A and US 5260440 B.

The dosage of rosuvastatin, a salt thereof, or a solvate of the compound or the salt is not particularly limited and can be determined by considering, for example, the gender, age and symptoms of the recipient as appropriate. However, for example, it is preferable that 0.5 to 80 mg, more preferably 1 to 60 mg, and particularly preferably 2.5 to 40 mg, of rosuvastatin in terms of its free form is taken once or two or more times per day.

Regarding the statin, from the viewpoint of obtaining excellent blood LDL cholesterol lowering effect when the statin is combined with the pyrimidine compound (1), a salt thereof, or a solvate of the compound or the salt, atorvastatin, pitavastatin, a salt thereof, or a solvate of the compound or the salt is preferred. Pitavastatin, a salt thereof, or a solvate of the compound or the salt is particularly preferred. As obvious from the Test Examples described below, in the case that the statins are used in combination with the pyrimidine compound (1), a salt thereof, or a solvate of the compound or the salt, a synergistic enhancement of the blood LDL cholesterol lowering effect is observed when pitavastatin, a salt thereof, or a solvate of the compound or the salt is used as the statins.

(S)-trans-{4-[({2-[({1-[3,5-bis(trifluoromethyl)phenyl]ethyl} {5-[2-(methylsulfonyl)ethoxy]pyrimidin-2-yl}amino)methyl]-4-(triflu oromethyl)phenyl}(ethyl)amino)methyl]cyclohexyl}acetic acid (pyrimidine compound (1)) is a known compound represented by Formula (1) described above, and can be produced by, for example, the methods disclosed in Patent Literature 5 and Patent Literature 6. The disclosures of these literatures are incorporated herein by reference.

The phrase "(S)-trans-{4-[({2-[({1-[3,5-bis(trifluoromethyl)phenyl]ethyl}{5-[2 -(methylsulfonyl)ethoxy]pyrimidin-2-yl}amino)methyl]-4-(trifluorome thyl)phenyl}(ethyl)amino)methyl]cyclohexyl}acetic acid, a salt thereof, or a solvate of the compound or the salt" according to the present specification means to include pyrimidine compound (1) itself, as well as a pharmaceutically acceptable salt of the pyrimidine compound (1), as well as a solvate of the pyrimidine compound (1) or a pharmaceutically acceptable salt thereof with water or an alcohol. The pharmaceutically acceptable salt is not particularly limited, and examples thereof include an acid addition salt or a base addition salt. Specifically, examples of the acid addition salt include acid addition salts with inorganic acids, such as a hydrochloride, a hydrobromide, a hydroiodide, a sulfate, a nitrate, and a phosphate; and acid addition salts with organic acids, such as a benzoate, a methanesulfonate, an ethanesulfonate, a benzenesulfonate, a p-toluenesulfonate, a maleate, a fumarate, a tartrate, a citrate, and an acetate. Furthermore, examples of the base addition salt include metal salts such as a sodium salt, a potassium salt, a lithium salt, a calcium salt, and a magnesium salt; salts with amines such as ammonia, trimethylamine, triethylamine, pyridine, cholidine, and lutidine; and base addition salts with organic bases such as lysine, arginine, cinchonine, and cinchonidine.

Regarding the salt of the pyrimidine compound (1), a hydrochloride is preferred from the viewpoint of obtaining

excellent blood LDL cholesterol lowering effect.

From another point of view, the salt of the pyrimidine compound (1) is preferably a hydrochloride (preferably, monohydrochloride), and more preferably a crystal of a hydrochloride (preferably, a crystal of monohydrochloride). It is considered that crystallization of the free form of the pyrimidine compound (1) is difficult. Thus, the applicant of the present invention found that when a hydrochloride among various salts is employed, a crystal having excellent thermal stability is obtained, and the applicant separately filed a patent application (International patent application (PCT/JP2014/064372) filed on May 30, 2014). The disclosure of this specification is incorporated herein by reference.

Therefore, in a case in which hydrochloride, particularly a crystal of hydrochloride, of the pyrimidine compound (1) is used, there is an advantage that a medicine having satisfactory thermal stability and satisfactory product quality can be obtained, and there is an advantage that stable and excellent blood LDL cholesterol lowering effect can be obtained. Furthermore, since the crystal of hydrochloride is in a solid form, there is also an advantage that handling at the time of producing a medicine, particularly a medicine in the form of a solid preparation, is easy.

In the following description, the hydrochloride of the pyrimidine compound (1) is described in detail.

In regard to the hydrochloride of the pyrimidine compound (1), the number of hydrochloric acid molecules is not particularly limited, and the hydrochloride may be any one of monohydrochloride, dihydrochloride, trihydrochloride, and tetrahydrochloride, or may also be a mixture of these. However, from the viewpoint of being obtainable as a stable acid addition salt, monohydrochloride is preferred.

Regarding the hydrochloride of the pyrimidine compound (1), (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid monohydrochloride represented by the following Formula (2):

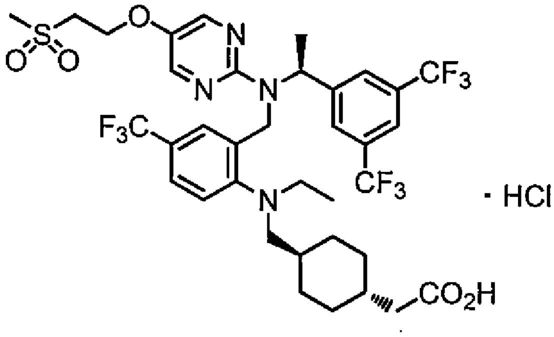

(2)

is preferred.

Regarding the crystal of the pyrimidine compound (1) hydrochloride, there are no particular limitations on the specific crystal type thereof, and any one of different crystal types may be employed, or a mixture of these crystal types is also acceptable. Furthermore, a mixture of the crystals with amorphous pyrimidine compound (1) hydrochloride is also acceptable.

Crystallinity of the pyrimidine compound (1) hydrochloride can be confirmed by, for example, known methods for determining crystallinity, such as X-ray diffraction analysis (specifically, for example, X-ray powder diffraction analysis), thermal analysis measurements (specifically, for example, differential thermal analysis (DTA) or differential scanning calorimetry (DSC)), confirmation of polarity (specifically, for example, observation made with a polarized microscope), and solid NMR analysis. For example, when a certain solid pyrimidine compound (1) hydrochloride is subjected to X-ray powder diffraction by irradiation with copper K$\alpha$ radiation, and in a case in which clear peaks are observed, the crystallinity of the pyrimidine compound (1) hydrochloride is confirmed. Meanwhile, the methods for determining crystallinity (for example, X-ray powder diffraction analysis and thermal analysis) can be carried out by referring to the descriptions in the Japanese Pharmacopeia, the US Pharmacopeia, and the European Pharmacopeia.

Furthermore, the confirmation of these crystals may also be carried out in the co-presence of other components. For example, in regard to a solid pharmaceutical composition (for example, tablets, capsules, granules, and powders) containing a hydrochloride of the pyrimidine compound (1) and a pharmaceutically acceptable carrier, the solid pharmaceutical composition is pulverized as needed, and an X-ray diffraction analysis is performed. In a case in which peaks originating from the hydrochloride of the pyrimidine compound (1) are observed, the crystallinity of the hydrochloride of the pyrimidine compound (1) is confirmed.

Regarding the crystal of the pyrimidine compound (1) hydrochloride, a crystal having peaks at one or more diffraction angles (2$\theta$) selected from the group consisting of at least the vicinity of 14.0 $\pm$ 0.2°, the vicinity of 18.3 $\pm$ 0.2°, the vicinity of 20.1 $\pm$ 0.2°, the vicinity of 20.5 $\pm$ 0.2°, the vicinity of 21.3 $\pm$ 0.2°, the vicinity of 21.8+0.2°, the vicinity of

23.3 ± 0.2°, and the vicinity of 24.0 ± 0.2°, is preferred in the X-ray powder diffraction pattern obtainable when the crystal is irradiated with copper Kα radiation. A crystal having a peak at a diffraction angle (2θ) at least in the vicinity of 20.5 ± 0.2° is more preferred. Also a crystal having peaks at diffraction angles (2θ) at least in the vicinity of 18.3 ± 0.2° and the vicinity of 20.5 ± 0.2° is more preferred. Also a crystal having peaks at diffraction angles (2θ) at least in the vicinity of 14.0 ± 0.2°, the vicinity of 18.3 ± 0.2°, the vicinity of 20.1 ± 0.2°, the vicinity of 20.5 ± 0.2°, the vicinity of 21.3 ± 0.2°, the vicinity of 21.8 ± 0.2°, the vicinity of 23.3 ± 0.2°, and the vicinity of 24.0 ± 0.2° is even more preferred. Also a crystal having a pattern that is substantially identical with the pattern illustrated in Fig. 1 is particularly preferred.

Also, from another point of view, regarding the crystal of the pyrimidine compound (1) hydrochloride, a crystal having an endothermic peak in the vicinity of about 162 ± 5°C in a differential thermal analysis (DTA) is preferred. Also a crystal which gives thermal analysis measurement (differential thermal analysis (DTA) and thermogravimetry (TG)) results that are substantially identical with the results illustrated in Fig. 2, is more preferred.

The hydrochloride of the pyrimidine compound (1) or a crystal thereof may be a solvate such as a hydrate, or may be a non-solvate such as an anhydride; however, an anhydride is preferred.

The hydrochloride of the pyrimidine compound (1) or a crystal thereof can be respectively produced by, for example, the following steps:

(Step 1) formation of a hydrochloride from the free form of the pyrimidine compound (1); and
(Step 2) formation of a crystal from a hydrochloride of the pyrimidine compound (1).

In the following description, the various steps will be respectively described in detail; however, the method for producing a hydrochloride of the pyrimidine compound (1) or a crystal thereof is not intended to be limited to the method described below.

<Step 1: Formation of hydrochloride from free form of pyrimidine compound (1) >

The present step is a step of bringing the pyrimidine compound (1) and hydrogen chloride together in the presence of a solvent, and thus forming a hydrochloride. Specifically, the present step is a step of dissolving the free form of the pyrimidine compound (1) in a solvent, supplying hydrogen chloride thereto, and performing salt formation.

In the present step, the free form of the pyrimidine compound (1) that serves as a starting raw material can be produced by, for example, the method described in Patent Literature 5.

The present step is carried out in the presence of a solvent. The solvent is not particularly limited as long as the solvent does not participate in the formation of hydrochloride. The examples of the solvent include aromatic hydrocarbons such as benzene, toluene, xylene, mesitylene, chlorobenzene, 1,2-dichlorobenzene, and nitrobenzene; ethers such as diethyl ether, diisopropyl ether, tert-butyl methyl ether, and 1,4-dioxane; acetic acid esters such as ethyl acetate, n-propyl acetate, and isopropyl acetate; ketones such as acetone, 2-butanone, and 3-pentanone; aliphatic hydrocarbons such as n-pentane, n-hexane, n-heptane, cyclohexane, n-octane, and n-decane; amides such as N,N-dimethylformamide and N,N-dimethylacetamide; and sulfoxides such as dimethyl sulfoxide. These can be used singly or in combination of two or more kinds thereof. In a case in which two or more kinds of solvents are used, these may be mixed, and then the pyrimidine compound (1) may be dissolved therein. Alternately, the pyrimidine compound (1) may be dissolved in one kind of solvent, and then the other solvent may be added thereto.

The solvent is preferably one or more selected from diisopropyl ether, tert-butyl methyl ether, 1, 4-dioxane, n-hexane, n-heptane, ethyl acetate, and isopropyl acetate; more preferably one or more selected from diisopropyl ether, tert-butyl methyl ether, 1,4-dioxane, and isopropyl acetate; and particularly preferably tert-butyl methyl ether or isopropyl acetate.

The amount of the solvent is not particularly limited; however, the solvent may be used in an amount, as a volume ratio, 1 to 20 times (V/W), and preferably 5 to 15 times (V/W), with respect to the weight of the free form of the pyrimidine compound (1).

The source of hydrogen chloride is not particularly limited, and in addition to blowing hydrogen chloride gas directly into the solution, for example, concentrated hydrochloric acid, a 4 M HCl/ethyl acetate solution, or a 4 M HCl/1,4-dioxane solution, all of which are easily available, can be utilized.

The amount of hydrogen chloride is not particularly limited; however, the amount with respect to the free form of the pyrimidine compound (1) is preferably 1 to 5 molar equivalents, and particularly preferably 1 to 4 molar equivalents.

The temperature for salt formation is not particularly limited; however, the temperature is usually in the range of -50°C to 150°C, preferably -20°C to 80°C, and more preferably -10°C to 40°C. The time required for salt formation is not particularly limited; however, the time is usually 5 minutes to 48 hours, preferably 30 minutes to 24 hours, and more preferably 30 minutes to 3 hours.

The hydrochloride of the pyrimidine compound (1) thus produced can be isolated. In this case, the salt precipitated as a solid may be isolated by a method that is usually used in the pertinent art, such as filtration, and may be dried

as necessary by a method that is usually used in the pertinent art. The drying means is not particularly limited, and drying under heating and/or under reduced pressure conditions may be employed. The drying temperature is preferably 50°C or lower, and more preferably 40°C to 50°C. The drying time is preferably 1 to 24 hours, and more preferably 6 to 12 hours.

<Step 2: Formation of crystal from hydrochloride of pyrimidine compound (1) >

The present step is a step of crystallizing the hydrochloride of the pyrimidine compound (1) (for example, amorphous form) obtained in Step 1, in the presence of a solvent. Specifically, the present step is a step of adding the hydrochloride of the pyrimidine compound (1) obtained in Step 1 in a solvent; dissolving the compound, for example, under heating as necessary; and then performing crystallization by, for example, cooling.

The present step is carried out in the presence of a solvent. Examples of the solvent include a mixed liquid of 2-propanol and heptane, and a mixed liquid of methyl ethyl ketone and heptane, and a mixed liquid of 2-propanol and heptane is preferred. The mixing ratio of the solvents is not particularly limited; however, heptane may be used in an amount, as a volume ratio, 0.1 to 2 times (V/V), and preferably 0.2 to 1 time (V/V), with respect to the volume of 2-propanol or methyl ethyl ketone.

In a case in which the hydrochloride of the pyrimidine compound (1) is dissolved in solvents, the solvents may be mixed in advance, and then the hydrochloride of the pyrimidine compound (1) may be dissolved therein. However, it is preferable that the hydrochloride of the pyrimidine compound (1) is dissolved in 2-propanol or methyl ethyl ketone, and then heptane is added thereto.

The amount of the solvent is not particularly limited; however, the solvent may be used in an amount, as a volume ratio, 1 to 20 times (V/W), and preferably 5 to 10 times (V/W), with respect to the weight of the hydrochloride of the pyrimidine compound (1), as the total amount of mixed solvent.

The temperature employed at the time of dissolving the hydrochloride of the pyrimidine compound (1) in a solvent is not particularly limited; however, the dissolving process may be carried out in a temperature range of usually 40°C to 100°C, and preferably 50°C to 80°C.

The temperature for crystallization of the hydrochloride of the pyrimidine compound (1) is not particularly limited; however, crystallization may be performed in a temperature range of usually 5°C to 40°C, preferably 10°C to 35°C, more preferably 10°C to 30°C, and particularly preferably 15°C to 25°C. In a case in which there is a gap between the temperature at the time of dissolving the hydrochloride of the pyrimidine compound (1) in solvents and the temperature of crystallization, cooling may be performed slowly for about 1 to 10 hours as appropriate, according to the temperature difference.

There are no particular limitations on the time required for crystallization; however, the time is usually one hour or longer, preferably 6 to 24 hours, and more preferably 8 to 16 hours.

The crystals of the pyrimidine compound (1) hydrochloride thus precipitated may be isolated by a method that is usually used in the pertinent art, such as filtration, and may be dried by a method that is usually used in the pertinent art as necessary. The drying means is not particularly limited, and drying under heating and/or under reduced pressure conditions may be employed. The drying temperature is preferably 50°C or lower, and more preferably 40°C to 50°C. The drying time is preferably 1 to 24 hours, and more preferably 6 to 12 hours.

Meanwhile, Step 2 may be carried out in the presence of crystals (seed crystals) of the pyrimidine compound (1) hydrochloride that have been produced separately. In this case, isopropyl acetate may be used as the solvent, instead of those described above. There are no particular limitations on the amount of the seed crystals; however, the seed crystals may be used in an amount of 0. 00001 to 0.05 parts by mass, and preferably 0.0001 to 0.01 parts by mass, with respect to the hydrochloride of the pyrimidine compound (1).

It is preferable that the seed crystals are added after the hydrochloride of the pyrimidine compound (1) has been dissolved in the solvent.

In the case of producing crystals of the pyrimidine compound (1) hydrochloride, from the viewpoint of simplifying the production process, the Step 1 and Step 2 are carried out in succession without isolating the hydrochloride of the pyrimidine compound (1). As a result, crystals of the hydrochloride can be produced from the free form of the pyrimidine compound (1) in the presence of a solvent. In this case, the solvent is preferably isopropyl acetate.

Furthermore, it is also possible to carry out Step 2 while omitting the time for salt formation in Step 1. That is, it is also possible that the free form of the pyrimidine compound (1) is dissolved in a solvent, hydrogen chloride is supplied thereto, the mixture is heated for example, and then crystallization is performed by, for example, cooling the mixture.

For example, the operation of other various processes is as described above.

The dosage of the pyrimidine compound (1), a salt thereof, or a solvate of the compound or the salt is not particularly limited and can be determined by considering, for example, the gender, age and symptoms of the recipient as appropriate. However, for example, it is preferable that about 0.01 to 1,000 mg, preferably 0.05 to 500 mg, and particularly preferably about 0.5 to 400 mg, of the compound in terms of the free form of the pyrimidine compound (1) is taken once or two or more times per day.

The combination ratio of a statin and the pyrimidine compound (1), a salt thereof, or a solvate of the compound or the salt is not particularly limited and can be determined by considering, for example, the gender, age and symptoms of the recipient as appropriate. However, from the viewpoint of obtaining excellent blood LDL cholesterol lowering effect, preferably 1 part by mass of a statin is used in combination with 0.01 to 1,000 parts by mass, more preferably with 0.05 to 500 parts by mass, and particularly preferably with 0.1 to 400 parts by mass, of the pyrimidine compound (1), a salt thereof, or a solvate of the compound or the salt in terms of the free form. Particularly, in a case in which pitavastatin, a salt thereof, or a solvate of the compound or the salt is used as the statin, from the viewpoint of obtaining excellent blood LDL cholesterol lowering effect, preferably 1 part by mass of pitavastatin, a salt thereof, or a solvate of the compound or the salt in terms of the free form is used in combination with 0.1 to 1,000 parts by mass, more preferably with 1 to 600 parts by mass, and particularly preferably 5 to 400 parts by mass, of the pyrimidine compound (1), a salt thereof, or a solvate of the compound or the salt in terms of the free form. Furthermore, in a case in which atorvastatin, a salt thereof, or a solvate of the compound or the salt is used as the statin, from the viewpoint of obtaining excellent blood LDL cholesterol lowering effect, preferably 1 part by mass of atorvastatin, a salt thereof, or a solvate of the compound or the salt in terms of the free form is used in combination with 0.01 to 500 parts by mass, more preferably with 0. 05 to 200 parts by mass, and particularly preferably 0.1 to 100 parts by mass, of the pyrimidine compound (1), a salt thereof, or a solvate of the compound or the salt in terms of the free form.

The "medicine for lowering LDL cholesterol" according to the present specification is preferably used in a case in which the amount of LDL cholesterol in blood is increasing (preferably, in a case in which the amount is increasing beyond the normal value) or in a case in which an increase in the amount of LDL cholesterol in blood is anticipated (for example, in a case in which although the amount of LDL cholesterol is temporarily being suppressed by taking medications, the amount of LDL cholesterol is expected to increase when the dosing of medications is stopped), in order to lower these increases.

The medicine for lowering LDL cholesterol can be used as, for example, a prophylactic and/or therapeutic agent for dyslipidemia (for example, primary hyperlipidemia or secondary hyperlipidemia), and preferably as a prophylactic and/or therapeutic agent for hypercholesterolemia (hyper-LDL-cholesterolemia) (for example, primary hypercholesterolemia, secondary hypercholesterolemia, or familial hypercholesterolemia).

In regard to the medicine for lowering LDL cholesterol comprising a statin and the pyrimidine compound (1), a salt thereof, or a solvate of the compound or the salt in combination, separate independent preparations each containing one of the components, namely, a statin, or the pyrimidine compound (1), a salt thereof, or a solvate of the compound or the salt (for example, in the form of a single package (kit preparation) comprising a combination of a preparation containing a statin and a preparation containing the pyrimidine compound (1), a salt thereof, or a solvate of the compound or the salt) may be produced. Those preparations may be administered simultaneously or with an interval. Alternatively, the medicine may also be administered as a pharmaceutical preparation containing both of the two components (combination drug). However, from the viewpoint of the convenience of dosing, it is preferable that the medicine is produced into a combination drug containing both of the two components.

The usage of a medicine for lowering LDL cholesterol containing the pyrimidine compound (1), a salt thereof, or a solvate of the compound or the salt, and being administered in combination with a statin, involves use of the medicine in combination with a statin, and for example, the medicine containing the pyrimidine compound (1), a salt thereof, or a solvate of the compound or the salt may be administered simultaneously with a medicine containing a statin, or with an interval between the two medicines.

A specific embodiment of the medicine may be, for example, a pharmaceutical product for lowering LDL cholesterol, the pharmaceutical product comprising the following (A) and (B):

(A) a medicine for lowering LDL cholesterol, the medicine containing the pyrimidine compound (1), a salt thereof, or a solvate of the compound or the salt; and
(B) an instruction sheet indicating that the medicine is administered in combination with a statin.

Specific examples of the instruction sheet include a so-called package leaflet (package insert) and a label, in which descriptions on, for example, the efficacy/effect and the usage/dosage are described.

Furthermore, the usage of a medicine for lowering LDL cholesterol containing a statin and being administered in combination with the pyrimidine compound (1), a salt thereof, or a solvate of the compound or the salt, involves use of the medicine in combination with the pyrimidine compound (1), and for example, a preparation containing a statin may be administered simultaneously with a preparation containing the pyrimidine compound (1), a salt thereof, or a solvate of the compound or the salt, or with an interval between the two preparations.

A specific embodiment of the medicine may be, for example, a pharmaceutical product for lowering LDL cholesterol, the pharmaceutical product comprising the following (A) and (B):

(A) a medicine for lowering LDL cholesterol, the medicine containing a statin; and

(B) an instruction sheet indicating that the medicine is administered in combination with the pyrimidine compound (1), a salt thereof, or a solvate of the compound or the salt.

Specific examples of the instruction sheet include a so-called package leaflet (package insert) and a label, in which descriptions on, for example, the efficacy/effect and the usage/dosage are described.

According to the present specification, specific form (dosage form) of the medicine is not particularly limited, and the medicine may have any form selected from a solid preparation, a semisolid preparation, or a liquid preparation, and can be selected according to, for example, the purpose of utilization of the medicine. Regarding the dosage form of the medicine, for example, the dosage forms described in the $16^{th}$ revision of the Japanese Pharmacopeia, General Rules for Preparations may be referred. More specifically, examples of the dosage form for oral administration include, for example, solid preparations such as tablets (for example, comprising ordinary tablets, intraorally disintegrable tablets, chewable tablets, effervescent tablets, dispersible tablets, and soluble tablets), capsules, granular formulations (for example, comprising effervescent granular formulations), and powders; semisolid preparations such as oral jelly formulations; and liquid preparations such as oral liquid formulations (for example, comprising elixirs, suspensions, emulsions, and limonades). Examples of the dosage form for parenteral administration include an injectable preparation, an inhalant, an eye drop, an ear drop, a nose drop, a suppository, an external solid formulation, an external liquid formulation, a spray preparation, an ointment, a cream preparation, a gel preparation, and a patch.

Regarding the dosage form of the medicine, from the viewpoint of ease of dosing, solid preparations for oral administration are preferred, and a tablet, a capsule, a granular formulation, or a powder is particularly preferred.

The medicine can be produced by a known method described in, for example, $16^{th}$ Revision of the Japanese Pharmacopeia General Rules for Preparations, depending on the dosage form. In this case, pharmaceutically acceptable carriers (additives) may be added to the medicine. Examples of such additives include an excipient, a binder, an extending agent, a disintegrant, a surfactant, a lubricating agent, a dispersant, a buffer agent, a preservative, a flavoring agent, a fragrance, a coating agent, and a diluent; however, the additives are not limited to these.

## Examples

Hereinafter, the present invention is specifically described by way of Examples, Test Examples, and Reference Examples; however, the present invention is not intended to be limited to these embodiments. In the following Examples, Test Examples, and Reference Examples, the free form of the pyrimidine compound (1) can be produced by the method described in Patent Literature 5. Furthermore, a hydrochloride of the pyrimidine compound (1) and a crystal thereof can be produced by the method described in the following Reference Example 1.

The abbreviations used in the Examples, Test Examples, and Reference Examples described below represent the following meanings.

| | |
|---|---|
| s: | singlet |
| d: | doublet |
| t: | triplet |
| q: | quartet |
| m: | multiplet |
| br: | broad |
| J: | coupling constant |
| Hz: | Hertz |
| DMSO-$d_6$: | Deuterated dimethyl sulfoxide |
| $^1$H-NMR: | proton nuclear magnetic resonance |

[Test Example 1] Evaluation of combined use of statin and CETP inhibitor

Test Example 1-1: Evaluation of combined use of pitavastatin and pyrimidine compound (1)

The presence or absence of an enhancement of the blood LDL cholesterol lowering effect through combined use of pitavastatin, a salt thereof, or a solvate of the compound or the salt and the pyrimidine compound (1), a salt thereof, or a solvate of the compound or the salt was evaluated by the following test.

1. Method

Male guinea pigs (six weeks old, Slc: Hartley, Japan SLC, Inc.) were used for the experiment. Blood was collected from the jugular vein, and the guinea pigs were grouped on the basis of the total plasma cholesterol level and

body weight. Starting from two days after the grouping, a solvent administration group (0.5% aqueous solution of methyl cellulose; control group), a pitavastatin single administration group, a pyrimidine compound (1) single administration group, and a pitavastatin-pyrimidine compound (1) combined administration group using were determined, and the various drugs and the solvent were orally administered for two weeks.

After completion of repeated administration, blood was collected under anesthesia with pentobarbital, and the amount of LDL cholesterol in blood plasma (mg/dl. Hereinafter, described as "amount of LDL cholesterol in blood plasma") was measured using high performance liquid chromatography according to the method by Usui, et al. (Usui et al., Clin. Chem., 46, 63-72 (2000)).

2. Group configuration

The group configuration consisted of the following four items, and six guinea pigs were used for each group.

(1) Control group
(2) Pitavastatin single administration group: Pitavastatin calcium pentahydrate was orally administered at a dose of 1 mg/kg of body weight.
(3) Pyrimidine compound (1) single administration group: Pyrimidine compound (1) monohydrochloride was orally administered at a dose of 30 mg/kg of body weight.
(4) Pitavastatin-pyrimidine compound (1) combined administration group: Pitavastatin calcium pentahydrate was orally administered at a dose of 1 mg/kg of body weight, and pyrimidine compound (1) monohydrochloride was orally administered at a dose of 30 mg/kg of body weight.

3. Statistical analysis and data processing

The amount of LDL cholesterol in blood plasma was obtained as an average value ± standard deviation. For the test results thus obtained, Dunnett's multiple comparison test was performed between the control group and the various drug administration groups, and thus the presence or absence of significant differences was determined (when the significance level was less than 5%, it was considered that there was a significant difference). Furthermore, a t-test was performed between the control group and the pyrimidine compound (1) single administration group, and the presence or absence of the significant difference was determined (when the significance level was less than 5%, it was considered that there was a significant difference). Furthermore, a t-test was performed between the pitavastatin single administration group and the pitavastatin-pyrimidine compound (1) combined administration group, and the presence or absence of the significant difference was determined (when the significance level was less than 5%, it was considered that there was a significant difference).

Furthermore, the proportions of lowering of LDL cholesterol in blood plasma in the various drug administration groups ("plasma LDL cholesterol lowering ratios") were calculated by the expression described below, from the average values of the amounts of LDL cholesterol in blood plasma of the control group and the various drug administration groups.

$$\text{Plasma LDL cholesterol lowering ratio (\%)} = [(Cc - Ct)/Cc] \times 100$$

wherein Cc represents the average value of the amount of LDL cholesterol in blood plasma in the control group; and Ct represents the average value of the amount of LDL cholesterol in blood plasma of each of the various drug administration groups.

Furthermore, the average values of the amounts of LDL cholesterol in blood plasma of each of the various drug administration groups in a case in which the average value of the amount of LDL cholesterol in blood plasma of the control group was designated as 1, were calculated as relative indices.

4. Test results

The test results are shown in Table 1.

[Table 1]

| Group | Amount of LDL cholesterol in blood plasma (mg/dl) | Plasma LDL cholesterol lowering ratio | Relative index |
|---|---|---|---|
| Control group | 22.4 ± 2.9 | - | - |
| Pitavastatin single administration group | 15.5 ± 3.9 | 31% | 0.69 |
| Pyrimidine compound (1) single administration group | 18.9 ± 3.8** | 16% | 0.84 |
| Pitavastatin-pyrimidine compound (1) combined administration group | 11.0 ± 2.8*** # | 51% | 0.49 |

In Table 1, the symbols ** and *** represent that there were significant differences at significance levels of less than 1% and less than 0.1%, respectively, in the comparison with the control group using Dunnett's multiple comparison test. The symbol # represents that there was a significant difference at a significance level of less than 5% in the comparison with the pitavastatin single administration group using a t-test.

Furthermore, determination of a synergistic effect according to Bulge formula was implemented.

From the test results shown in Table 1, a significant decrease in the amount of LDL cholesterol in blood plasma was recognized in the pitavastatin-pyrimidine compound (1) combined administration group, as compared to the control group and the pitavastatin single administration group. The proportion of the amount of LDL cholesterol in blood plasma compared to the control group (relative index) was 0.69 for the pitavastatin single administration group, and 0.84 for the pyrimidine compound (1) single administration group, the product thereof (0.58) was larger than the relative index 0.49 of the combined administration group. Thus, a synergistic effect was confirmed by Bulge formula.

From the test results as described above, it became clear that when pitavastatin, a salt thereof, or a solvate of the compound or the salt and the pyrimidine compound (1), a salt thereof, or a solvate of the compound or the salt are administered in combination, the plasma LDL cholesterol lowering effect is synergistically enhanced.

Test Example 1-2: Evaluation of combined use of pitavastatin and anacetrapib

A test was carried out by a method that was completely the same as that used in Test Example 1-1, except that anacetrapib was administered at a dose of 30 mg/kg of body weight instead of 30 mg/kg of body weight of the pyrimidine compound (1).

The results are shown in Table 2.

[Table 2]

| Drug tested | Amount of LDL cholesterol in blood (mg/dl) | Plasma LDL cholesterol lowering ratio | Relative index |
|---|---|---|---|
| Control group | 18.5 ± 4.8 | - | - |
| Pitavastatin single administration group | 11.5 ± 3.3 | 38% | 0.62 |
| Anacetrapib single administration group | 18.2 ± 4.1 | 2% | 0.98 |
| Pitavastatin-anacetrapib combined administration group | 13.1 ± 2.9 | 29% | 0.71 |

According to the test results shown in Table 2, in a case when anacetrapib was used as a CETP inhibitor, unlike the case of using the pyrimidine compound (1), a further decrease in the amount of LDL cholesterol in blood plasma was not observed compared to the pitavastatin single administration group, and also an enhancement of the plasma LDL cholesterol lowering effect due to combined use was not recognized.

From the results of Test Examples 1-1 and 1-2 described above, it became clear that combined use of a statin and the pyrimidine compound (1), a salt thereof, or a solvate of the compound or the salt exhibited excellent plasma LDL cholesterol lowering effect, and also an enhancement of the plasma LDL cholesterol lowering effect due to the combined use is markedly strong compared to that of the combined use of a statin and anacetrapib.

[Reference Test Example 1] Investigation of conditions for crystallization

For various samples (free form of the pyrimidine compound (1), and salts thereof (hydrochloride, hydrobromide, sulfate, D-(-)-arginine salt, and cinchonidine salt), the conditions for crystallization were investigated by the following methods.

Meanwhile, hydrochloride of the pyrimidine compound (1) was obtained by the method described in Step 1 of Reference Example 1, 1-1. The other salts were obtained by mixing and stirring an equimolar mixture of the free form of the pyrimidine compound (1) dissolved in methanol and an acid or a base dissolved in water, and then distilling off the solvent.

Each of various samples was dissolved in Solvent 1 in an amount 4 to 100 times (V/W) the amount of the sample. Solvent 2 was added to the solution thus obtained, until the solution became cloudy, and then the mixture was subjected to a predetermined operation. Subsequently, the solution state was observed by visual inspection, and the presence or absence of crystal formation was evaluated.

The results are shown in Table 3 for the free form of the pyrimidine compound (1), in Table 4 for the hydrochloride, in Table 5 for the hydrobromide, in Table 6 for the sulfate, in Table 7 for the D-(-)-arginine salt, and in Table 8 for the cinchonidine salt.

[Table 3]

| Investigation on crystallization of free form of pyrimidine compound (1) | | | |
|---|---|---|---|
| Solvent 1 | Solvent 2 | Operation | Observation results |
| 2-Propanol | Water | Hermetically sealed and left to stand at room temperature for 72 hours | Oil |
| Acetone | Water | Hermetically sealed and left to stand at room temperature for 72 hours | Separated into two layers |
| Dichloromethane | Heptane | Left to stand at room temperature for 72 hours | Oil |
| Ethanol | Heptane | Left to stand at room temperature for 72 hours | Oil |
| 2-Propanol | Heptane | Left to stand at room temperature for 72 hours | Oil |
| Ethyl acetate | Heptane | Left to stand at room temperature for 72 hours | Oil |
| Methyl ethyl ketone | Heptane | Left to stand at room temperature for 72 hours | Oil |
| Toluene | Heptane | Left to stand at room temperature for 72 hours | Oil |

[Table 4]

| Investigation on crystallization of hydrochloride of pyrimidine compound (1) | | | |
|---|---|---|---|
| Solvent 1 | Solvent 2 | Operation | Observation results |
| Ethanol | Toluene | Hermetically sealed and left to stand at room temperature for 96 hours | Homogeneous solution |
| Ethanol | Water | Hermetically sealed and left to stand at room temperature for 96 hours | Separated into two layers |
| 2-Propanol | Heptane | Hermetically sealed and left to stand at room temperature for 96 hours | Crystal precipitated |
| Methyl ethyl ketone | Heptane | Hermetically sealed and left to stand at room temperature for 96 hours | Crystal precipitated |
| Dichloromethane | Heptane | Hermetically sealed and left to stand at room temperature for 96 hours | Oil |
| Tetrahydrofuran | Toluene | Left to stand at room temperature for 96 hours | Oil |
| 1,4-Dioxane | tert-Butyl methyl ether | Left to stand at room temperature for 72 hours | Oil |

(continued)

| Investigation on crystallization of hydrochloride of pyrimidine compound (1) | | | |
|---|---|---|---|
| Solvent 1 | Solvent 2 | Operation | Observation results |
| Dichloromethane | tert-Butyl methyl ether | Left to stand at room temperature for 72 hours | Oil |

[Table 5]

| Investigation on crystallization of hydrobromide of pyrimidine compound (1) | | | |
|---|---|---|---|
| Solvent 1 | Solvent 2 | Operation | Observation results |
| 2-Propanol | Toluene | Left to stand at room temperature for 96 hours | Oil |
| Ethyl acetate | Toluene | Left to stand at room temperature for 96 hours | Oil |
| 2-Propanol | Heptane | Left to stand at room temperature for 72 hours | Oil |
| 1,4-Dioxane | Heptane | Left to stand at room temperature for 72 hours | Oil |
| Methyl ethyl ketone | Heptane | Left to stand at room temperature for 72 hours | Oil |
| Dichloromethane | tert-Butyl methyl ether | Left to stand at room temperature for 72 hours | Oil |
| 1,4-Dioxane | tert-Butyl methyl ether | Left to stand at room temperature for 72 hours | Oil |
| Acetone | tert-Butyl methyl ether | Left to stand at room temperature for 72 hours | Oil |

[Table 6]

| Investigation on crystallization of sulfate of pyrimidine compound (1) | | | |
|---|---|---|---|
| Solvent 1 | Solvent 2 | operation | Observation results |
| 2-Propanol | Toluene | Left to stand at room temperature for 96 hours | Oil |
| Tetrahydrofuran | Toluene | Left to stand at room temperature for 96 hours | Oil |
| 2-Propanol | Heptane | Left to stand at room temperature for 96 hours | Oil |
| Methyl ethyl ketone | Heptane | Left to stand at room temperature for 72 hours | Oil |
| Ethyl acetate | Heptane | Left to stand at room temperature for 72 hours | Oil |
| 1,4-Dioxane | Heptane | Left to stand at room temperature for 72 hours | Oil |
| 2-Propanol | tert-Butyl methyl ether | Left to stand at room temperature for 72 hours | Oil |
| Acetonitrile | tert-Butyl methyl ether | Left to stand at room temperature for 72 hours | Oil |

[Table 7]

| Investigation on crystallization of D- (-) -arginine salt of pyrimidine compound (1) | | | |
|---|---|---|---|
| Solvent 1 | Solvent 2 | Operation | Observation results |
| Methanol | Water | Left to stand at room temperature for 2 days | Gelatinous substance |
| Ethanol | Toluene | Left to stand at room temperature for 2 days | Homogeneous solution |
| Ethanol | Heptane | Left to stand at room temperature for 2 days | Homogeneous solution |
| Acetonitrile | tert-Butyl methyl ether | Left to stand at room temperature for 2 days | Oil |
| Ethyl acetate | Toluene | Left to stand at room temperature for 2 days | Oil |

(continued)

| Investigation on crystallization of D- (-) -arginine salt of pyrimidine compound (1) | | | |
|---|---|---|---|
| Solvent 1 | Solvent 2 | Operation | Observation results |
| Ethyl acetate | Heptane | Left to stand at room temperature for 2 days | Oil |
| Tetrahydrofuran | Water | Left to stand at room temperature for 2 days | Oil |
| Tetrahydrofuran | Heptane | Left to stand at room temperature for 2 days | Oil |

[Table 8]

| Investigation on crystallization of cinchonidine salt of pyrimidine compound (1) | | | |
|---|---|---|---|
| Solvent 1 | Solvent 2 | Operation | Observation results |
| Methanol | - | Left to stand at room temperature for 2 days | Homogeneous solution |
| Ethanol | - | Left to stand at room temperature for 2 days | Homogeneous solution |
| 2-Propanol | - | Left to stand at room temperature for 2 days | Homogeneous solution |
| Tetrahydrofuran | - | Left to stand at room temperature for 2 days | Homogeneous solution |
| Chloroform | - | Left to stand at room temperature for 2 days | Homogeneous solution |
| Ethyl acetate | - | Left to stand at room temperature for 2 days | Homogeneous solution |
| Acetone | - | Left to stand at room temperature for 2 days | Homogeneous solution |
| tert-Butyl methyl ether | - | Left to stand at room temperature for 2 days | Homogeneous solution |

From the investigation results as described above, it became clear that the pyrimidine compound (1) is precipitated into crystals specifically in a case in which hydrochloride of the compound is used.

[Reference Example 1] Production of crystals of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride

1-1: Production of crystals of pyrimidine compound (1) hydrochloride, Mode 1

Step 1

1.1 kg (1.35 mol) of the free form of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid was dissolved in tert-butyl methyl ether (15.3 kg) in an argon atmosphere, and the solution was cooled to 0°C. Next, 503.9 g of a 16.7% hydrochloric acid/1,4-dioxane solution (hydrochloric acid 2.31 mol) was added dropwise to the solution thus obtained at 0°C to 10°C, and then the mixture was stirred for one hour at the same temperature. A solid thus precipitated was collected by filtration, subsequently washed with chilled tert-butyl methyl ether (1.85 kg), and dried under reduced pressure for 12 hours at 40°C to 50°C. Thus, 1.14 kg (yield 100%) of amorphous (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride was obtained.

$^1$H-NMR(400MHz,DMSO-$d_6$)$\delta$:0.72-0.92(7H,m), 1.30(1H,m), 1.50(3H,d,J=6.6H z), 1.62-1.70(5H,m), 2.02(2H,d,J=6.8Hz), 2.71(1H,m), 2.75(1H,brs), 2.90( 3H,brs), 3.07(3H,s), 3.62(2H,t,J=5.5Hz), 4.40(2H,t,J=5.7Hz), 4.67(1H,d, J=17.6Hz), 4.80(1H,d,J=17.8Hz), 6.24(1H,q,J=6.8Hz), 7.10(1H,s), 7.33(1H ,brs), 7.47(1H,d,J=8.3Hz), 7.84(2H,s), 7.94(1H,s), 8.35(2H,s).

Step 2

The amorphous (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride (676 mg) obtained in Step 1 was dissolved in 2-propanol (1.35 mL) under heating at 50°C to 55°C, and then heptane (676 μL) was

added thereto at 50°C. The mixture was left to stand hermetically for 14 hours at 5°C to 15°C. A solid thus precipitated was collected by filtration, and was dried under reduced pressure at 40°C. Thus, 576 mg (yield 85%) of crystals of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride were obtained.

1-2: Production of crystals of pyrimidine compound (1) hydrochloride, Mode 2

1.14 kg (1.35 mol) of the amorphous (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride obtained by the method described in Section 1-1, Step 1 was suspended in isopropyl acetate (9.98 kg), and the suspension was dissolved under heating to 65°C to 75°C. 11 g of the crystals of (S)-trans-{4-[({2-[({1-[3,5-bis(trifluoromethyl)phenyl]ethyl}{5-[2-(methylsulfonyl)ethoxylpyrimidin-2-yl}amino)methyl]-4-(trifluoromet hyl)phenyl}(ethyl)amino)methyl]cyclohexyl}acetic acid hydrochloride obtained by the method described in Section 1-1, Step 2 were added to the solution thus obtained as seed crystals at the same temperature, and the mixture was stirred for 3 hours. Subsequently, the mixture was cooled to 45°C to 55°C over 2 hours, further cooled to 15°C to 25°C over 3 hours, and stirred for another 16 hours at the same temperature. Crystals thus precipitated were collected by filtration, washed with isopropyl acetate (1720 g), and dried under reduced pressure for 12 hours at 35°C to 45°C. Thus, 1.02 kg (yield 85%) of crystals of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride were obtained.

As a result of an elemental analysis, it became clear that the hydrochloride thus obtained was monohydrochloride as follows.

Elemental analysis results:

Calculated values (as monohydrochloride): C 50.91%, H 4.98%, N 6.60%, Cl 4.17%
Measured values: C 50.79%, H 4.70%, N 6.40%, Cl 3.94%

$^1$H-NMR(400MHz,DMSO-$d_6$)$\delta$:0.72-0.92(7H,m), 1.29(1H,m), 1.49(3H,d,J=6.8H z), 1.62-1.70(5H,m), 2.02(2H,d,J=6.6Hz), 2.71(1H,m), 2.80-2.90(3H,m), 3. 07(3H,s), 3.62(2H,t,J=5.5Hz), 4.40(2H,t,J=5.7Hz), 4.65(1H,d,J=16.4Hz), 4.78(1H,d,J=17.1Hz), 6.23(1H,q,J=6.8Hz), 7.09(1H,s), 7.29(1H,d,J=8.0Hz ), 7.45(1H,d,J=7.8Hz), 7.83(2H,s), 7.94(1H,s), 8.35(2H,s).

1-3: Production of crystals of pyrimidine compound (1) hydrochloride, Mode 3

16.1 kg (19.8 mol) of the free form of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl)acetic acid was dissolved in isopropyl acetate (124 kg) in an argon atmosphere, and the solution was heated to 40°C to 50°C. Next, 15.0 kg of a 6.3% hydrochloric acid/isopropyl acetate solution (hydrochloric acid 25.98 mol) was added dropwise to the solution thus obtained, and then the mixture was heated to 65°C to 75°C. To the solution thus obtained, 25 g of the crystals of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride obtained by the method described in Section 1-1, Step 2 were added as seed crystals at the same temperature. Furthermore, 7.0 kg of a 6.3% hydrochloric acid/isopropyl acetate solution (hydrochloric acid 12.08 mol) was added dropwise thereto, and the resulting mixture was stirred for 7 hours. Subsequently, the mixture was cooled to 45°C to 55°C over 3 hours, further cooled to 15°C to 25°C over 4 hours, and stirred for another 16 hours at the same temperature. Crystals thus precipitated were collected by filtration, subsequently washed with isopropyl acetate (32.4 kg), and dried under reduced pressure for 12 hours at 40°C to 50°C. Thus, 15.35 kg (yield 91%) of crystals of (S)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride were obtained.

As a result of an elemental analysis, it became clear that the hydrochloride thus obtained was monohydrochloride.

Elemental analysis results:

Calculated values (as monohydrochloride): C 50.91%, H 4.98%, N 6.60%, Cl 4.17%

Measured values: C 50.82%, H 4.98%, N 6.56%, Cl 4.15%

$^1$H-NMR(400MHz,DMSO-$d_6$)δ:0.72-0.92(7H,m), 1.29(1H, m), 1.49(3H,d,J=6.8H z), 1.62-1.70(5H,m), 2.02(2H,d,J=6.6Hz), 2.71(1H,m), 2.80-2.90(3H,m), 3. 07(3H,s), 3.62(2H,t,J=5.5Hz), 4.40(2H,t,J=5.7Hz), 4.65(1H,d,J=16.4Hz), 4.78(1H,d,J=17.1Hz), 6.23(1H,q,J=6.8Hz), 7.09(1H,s), 7.29(1H,d,J=8.0Hz ), 7.45(1H,d,J=7.8Hz), 7.83(2H,s), 7.94(1H,s), 8.35(2H,s).

1-4: Evaluation of properties of crystals of pyrimidine compound (1) hydrochloride

For the crystals obtained in Section 1-3 described above, X-ray powder diffraction measurement and thermal analysis measurement were carried out as described below.

<X-ray powder diffraction measurement>

The crystals obtained in Section 1-3 described above were subjected to X-ray powder diffraction measurement. The X-ray powder diffraction measurement was performed under the following conditions, by filling a sample holder part of a non-reflective silicon sample plate for X-ray diffraction with a pulverized crystal sample.

X-ray powder diffraction analyzer: RINT-Ultima IV-Protectus (manufactured by Rigaku Corp.)
X-ray type: copper Kα radiation (λ = 1.54 A)
Scan range for diffraction angle 2θ: 3.00° to 40.00°
Sampling width: 0.02°
Scan speed: 2.00°/min

The diffraction pattern thus obtained is shown in Fig. 1. In Fig. 1, the vertical axis represents the diffraction intensity (counts/second (cps)), and the horizontal axis represents the diffraction angle 2θ (°).

Furthermore, for major peaks having a relative intensity of 30 or higher, the diffraction angle 2θ, the half peak width, d value, intensity, and relative intensity are shown in Table 9.

From Fig. 1 and Table 9, it became clear that the pattern shows major peaks at diffraction angles (2θ) in the vicinity of 14.0 ± 0.2°, the vicinity of 18.3 ± 0.2°, the vicinity of 20.1 ± 0.2°, the vicinity of 20.5 ± 0.2°, the vicinity of 21.3 ± 0.2°, the vicinity of 21.8 ± 0.2°, the vicinity of 23.3 ± 0.2°, and the vicinity of 24.0 ± 0.2°.

Furthermore, it became clear that the pattern has peaks with high intensity at diffraction angles (2θ) in the vicinity of 18.3 ± 0.2° and the vicinity of 20.5 ± 0.2°, and particularly at a diffraction angle (2θ) in the vicinity of 20.5 ± 0.2°.

[Table 9]

| Peak number | 2θ | Half peak width | d value | Intensity | Relative intensity |
|---|---|---|---|---|---|
| 1 | 14.000 | 0.235 | 6.3205 | 1232 | 31 |
| 2 | 18.260 | 0.235 | 4.8545 | 2486 | 62 |
| 3 | 20.100 | 0.235 | 4.4140 | 1279 | 32 |
| 4 | 20.500 | 0.235 | 4.3288 | 4040 | 100 |
| 5 | 21.340 | 0.235 | 4.1603 | 1194 | 30 |
| 6 | 21.780 | 0.235 | 4.0772 | 1386 | 35 |
| 7 | 23.320 | 0.235 | 3.8113 | 1415 | 36 |
| 8 | 23.960 | 0.212 | 3.7109 | 1421 | 36 |

<Thermal analysis measurement>

The crystals obtained in Reference Example 1-3 were subjected to thermal analysis measurement. The thermal analysis measurement was carried out by differential thermal analysis (DTA) and thermogravimetry (TG) using a thermal analysis apparatus, THERMO PLUS 2 system (manufactured by Rigaku Corp.), after precisely weighing about 5 mg of a sample in an aluminum pan for thermal analysis, using $Al_2O_3$ as a reference substance, in a nitrogen atmosphere (150 mL/min) at a rate of temperature increase of 10°C/min.

The results for the thermal analysis measurement are illustrated in Fig. 2. In Fig. 2, the vertical axis represents the thermoelectromotive force (μV) of a thermocouple in the DTA curve and represents mass change (mg) in the TG curve, and the horizontal axis represents temperature (°C).

As can be seen from Fig. 2, the crystals of the pyrimidine compound (1) hydrochloride had an endothermic peak near 162 ± 5°C (specifically, 161.6°C) in the differential thermal analysis (DTA). From the results of the thermal analysis measurement, it was considered that the crystals of the pyrimidine compound (1) hydrochloride have a melting point at near 162 ± 5°C.

[Reference Test Example 2] Thermal stability test

A test compound was introduced into glass bottles, and the bottles were stored for certain time periods under temperature conditions of 80°C, 100°C, or 120°C. Subsequently, the residual ratio (%) of the pyrimidine compound (1) in the test compound was measured.
Regarding the residual ratio, the proportion of the pyrimidine compound (1) contained in the test compound was measured as the peak area percentage using high performance liquid chromatography. In regard to the measurement by high performance liquid chromatography, an ODS column was used as a column, and a mixture of two kinds, namely, a 0.1% aqueous solution of TFA and a 0.1% acetonitrile solution of TFA, was used as a solvent. The detection wavelength was 242 nm.
The residual ratio was calculated by the following calculation expression from the area percentage of the pyrimidine compound (1) thus obtained.
Residual ratio (%) = Area percentage of pyrimidine compound (1) after storage/area percentage of pyrimidine compound (1) before storage × 100
As the test compound, the crystals (crystals of pyrimidine compound (1) hydrochloride) obtained in Reference Example 1-3, and the free form of the pyrimidine compound (1) were used.
The results are shown in Table 10.

[Table 10]

| | Residual ratio (%) | | |
|---|---|---|---|
| | After storage at 80°C for 72 hours | After storage at 100°C for 24 hours | After storage at 120°C for 16 hours |
| Crystals of pyrimidine compound (1) hydrochloride | 100.0 | 100.0 | 99.5 |
| Free form of pyrimidine compound (1) | 99.5 | 89.3 | 71.5 |

It became clear from the test results described in Table 10 that the crystals of pyrimidine compound (1) hydrochloride had excellent thermal stability.

Industrial Applicability

According to the present invention, since a medicine having excellent blood LDL cholesterol lowering effect can be provided, the medicine can be utilized in, for example, the pharmaceutical industry.

## Claims

1. A medicine for lowering LDL cholesterol, the medicine comprising a combination of a statin and (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

2. The medicine according to claim 1, wherein the medicine is in the form of a combination drug containing both a statin and (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

3. The medicine according to claim 1, wherein the medicine is in the form of a kit preparation comprising a combination of a preparation containing a statin and a preparation containing (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

**4.** A medicine for lowering LDL cholesterol, the medicine comprising (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, and being administered in combination with a statin for the purpose of lowering LDL cholesterol.

**5.** A medicine for lowering LDL cholesterol, the medicine comprising a statin, and being administered for the purpose of lowering LDL cholesterol in combination with (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl} (ethyl) amino)methyl]cyclohexyl}acetic acid, a salt thereof, or a solvate of the compound or the salt.

**6.** The medicine according to any one of claims 1 to 5, wherein the statin is one or more selected from the group consisting of lovastatin, pravastatin, simvastatin, fluvastatin, atorvastatin, pitavastatin, rosuvastatin, salts thereof, and solvates of the compounds and the salts.

**7.** The medicine according to any one of claims 1 to 5, wherein the statin is pitavastatin, a salt thereof, or a solvate of the compound or the salt.

**8.** The medicine according to any one of claims 1 to 7, wherein (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl} (ethyl)amino)methyl]cyclohexyl}acetic acid, a salt thereof, or a solvate of the compound or the salt is (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride.

**9.** The medicine according to claim 8, wherein the (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride is in the form of crystals.

**10.** The medicine according to any one of claims 1 to 9, wherein the medicine is a prophylactic and/or therapeutic agent for dyslipidemia.

**11.** The medicine according to any one of claims 1 to 9, wherein the medicine is a prophylactic and/or therapeutic agent for hypercholesterolemia.

**12.** Use of a combination of a statin and (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the production of a medicine for lowering LDL cholesterol.

**13.** Use of (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the production of a medicine for lowering LDL cholesterol, the medicine being administered in combination with a statin.

**14.** Use of a statin for the production of a medicine for lowering LDL cholesterol, the medicine being administered in combination with (*S*)-trans-{4-[({2-[({l-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt.

**15.** The use according to any one of claims 12 to 14, wherein the statin is one or more selected from the group consisting of lovastatin, pravastatin, simvastatin, fluvastatin, atorvastatin, pitavastatin, rosuvastatin, salts thereof, and solvates of the compounds and the salts.

**16.** The use according to any one of claims 12 to 14, wherein the statin is pitavastatin, a salt thereof, or a solvate of the compound or the salt.

**17.** The use according to any one of claims 12 to 16, wherein (*S*)-trans-{4-[({2-[({1-[3,5-bis(trifluoromethyl)phenyl]

ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof , or a solvate of the compound or the salt is (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride.

**18.** The use according to claim 17, wherein (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride is in the form of crystals.

**19.** The use according to any one of claims 12 to 18, wherein the medicine is a prophylactic and/or therapeutic agent for dyslipidemia.

**20.** The use according to any one of claims 12 to 18, wherein the medicine is a prophylactic and/or therapeutic agent for hypercholesterolemia.

**21.** A combination of a statin and (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}{ethyl)amino)methyl]cyclohexyl}acetic acid, a salt thereof, or a solvate of the compound or the salt, for lowering LDL cholesterol.

**22.** (*S*)-trans-{4-[({2-[({1-[3,5-bis(trifluoromethyl)phenyl]ethyl} {5-[2-(methylsulfonyl)ethoxy]pyrimidin-2-yl}amino)methyl]-4-(triflu oromethyl)phenyl}(ethyl)amino)methyl]cyclohexyl}acetic acid, a salt thereof, or a solvate of the compound or the salt, for being administered in combination with a statin for the purpose of lowering LDL cholesterol.

**23.** A statin for being administered in combination with (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt, for the purpose of lowering LDL cholesterol.

**24.** The compound, a salt thereof, or a solvate of the compound or the salt according to any one of claims 21 to 23, wherein the statin is one or more selected from the group consisting of lovastatin, pravastatin, simvastatin, fluvastatin, atorvastatin, pitavastatin, rosuvastatin, salts thereof, and solvates of the compounds and the salts.

**25.** The compound, a salt thereof, or a solvate of the compound or the salt according to any one of claims 21 to 23, wherein the statin is pitavastatin, a salt thereof, or a solvate of the compound or the salt.

**26.** The compound, a salt thereof, or a solvate of the compound or the salt according to any one of claims 21 to 25, wherein (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid, a salt thereof, or a solvate of the compound or the salt is (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride.

**27.** The compound, a salt thereof, or a solvate of the compound or the salt according to claim 26, wherein (*S*)-trans-{4-[({2-[({1-[3,5-bis(trifluoromethyl)phenyl]ethyl}{5-[2-(methylsulfonyl)ethoxy]pyrimidin-2-yl}amino)methyl]-4-(trifluoromet hyl)phenyl}(ethyl)amino)methyl]cyclohexyl}acetic acid hydrochloride is in the form of crystals.

**28.** The compound, a salt thereof, or a solvate of the compound or the salt according to any one of claims 21 to 27, for use in preventing and/or treating dyslipidemia.

**29.** The compound, a salt thereof, or a solvate of the compound according to any one of claims 21 to 27, for use in preventing and/or treating hypercholesterolemia.

Figure 1

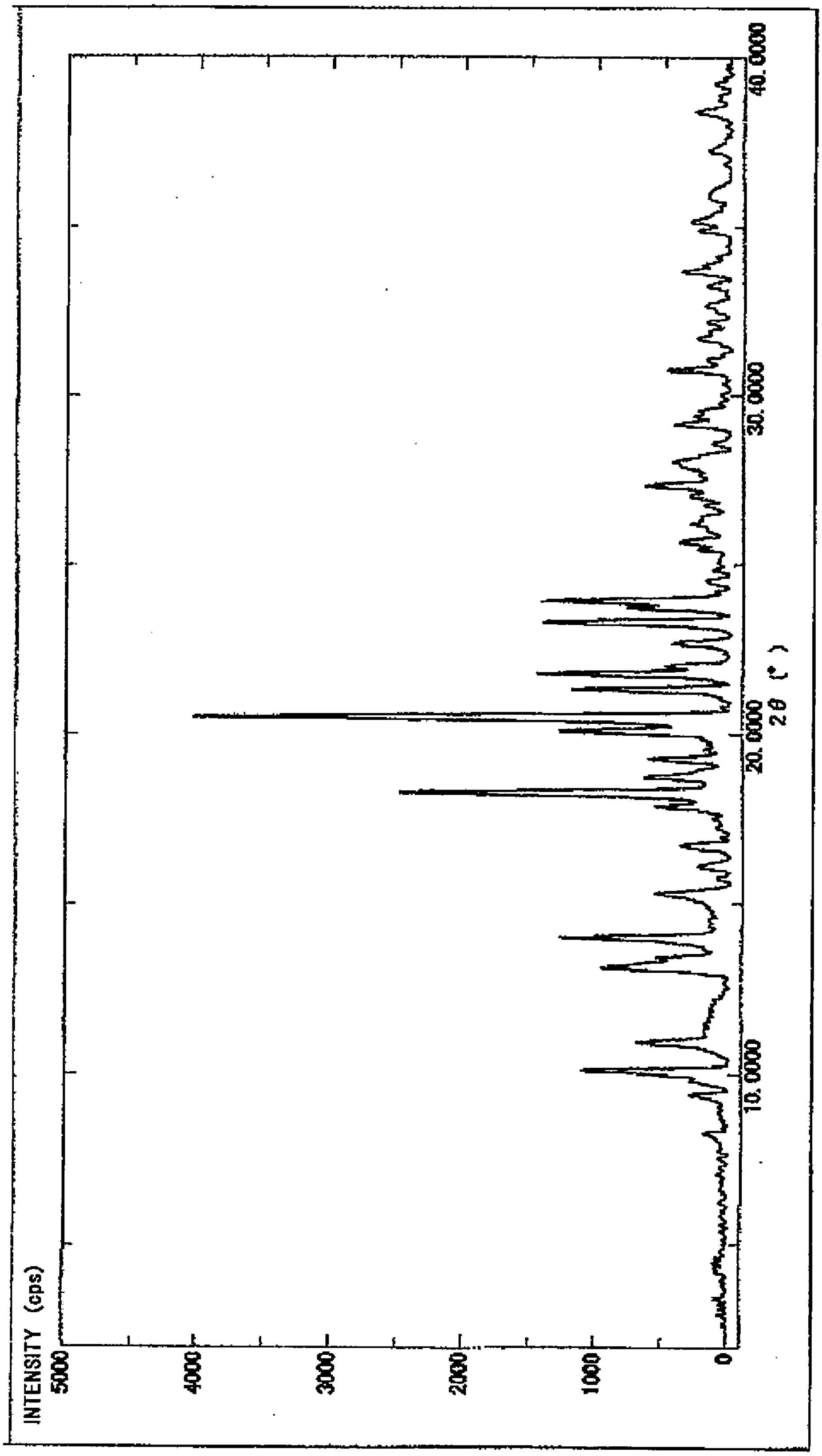
INTENSITY (cps)
5000
4000
3000
2000
1000
0
10.0000
20.0000
30.0000
40.0000
2θ (°)

Figure 2

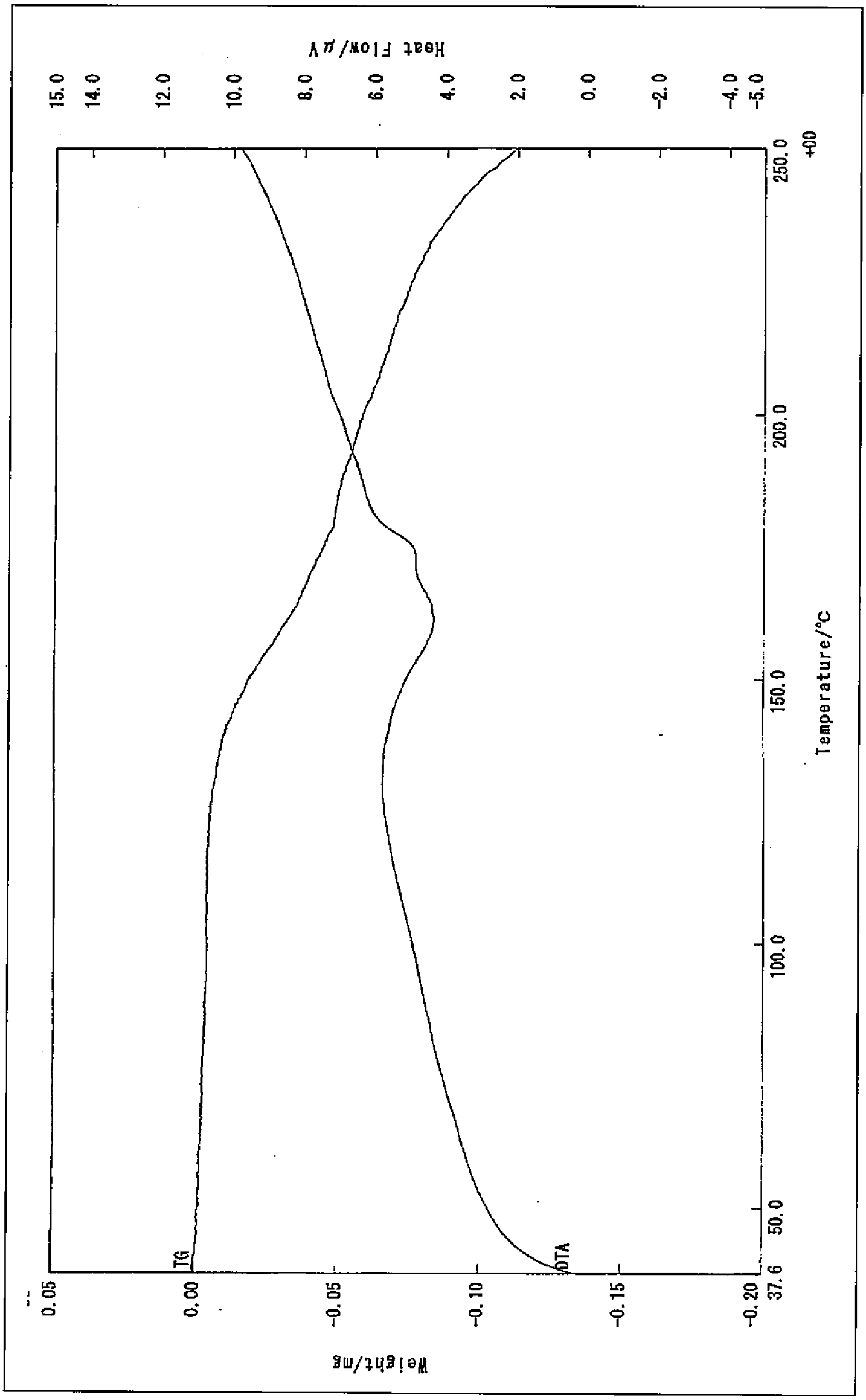
Heat Flow/μV
15.0
14.0
12.0
10.0
8.0
6.0
4.0
2.0
0.0
-2.0
-4.0
-5.0
Weight/mg
0.05
0.00
-0.05
-0.10
-0.15
-0.20
Temperature/°C
37.6
50.0
100.0
150.0
200.0
250.0
+00
TG
DTA

## INTERNATIONAL SEARCH REPORT

International application No.
PCT/JP2015/083452

A. CLASSIFICATION OF SUBJECT MATTER
*A61K31/505*(2006.01)i, *A61K31/47*(2006.01)i, *A61K45/00*(2006.01)i, *A61P3/06*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K31/00-31/80, A61P1/00-43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2014-520795 A (Novartis AG.), 25 August 2014 (25.08.2014), claims; paragraphs [0119], [0237] to [0241], [0261]; example 57A & WO 2013/008164 A2 claims; page 35, right middle part; page 69, 1st to 3rd paragraphs; page 75, 3rd paragraph & US 2014/0134262 A1 & EP 2729142 A2 & CA 2841117 A & CN 103648495 A & KR 10-2014-0056258 A & RU 2014104377 A | 1-7,10-17, 19-22,24-29<br>8,9,18 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 January 2016 (25.01.16) | Date of mailing of the international search report<br>09 February 2016 (09.02.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.
PCT/JP2015/083452

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2011/152508 A1 (Kowa Co., Ltd.), 08 December 2011 (08.12.2011), claims; paragraphs [0048], [0109], [0135]<br>& EP 2578574 A1<br>paragraphs [0048], [0110], [0135]<br>& US 8906895 B2 & CN 102933559 A<br>& MX 2012014149 A & KR 10-2013-0111512 A<br>& TW 201208688 A | 22,24-29<br>1-21 |
| Y | WO 2013/063217 A1 (Merck Sharp & Dohme Corp.), 02 May 2013 (02.05.2013), page 14, line 36 to page 15, line 3<br>& JP 2014-532652 A<br>paragraph [0120]<br>& US 2014/0378493 A1 & EP 2771345 A1<br>& AU 2012328759 A & CA 2852743 A<br>& CN 103958524 A & KR 10-2014-0094571 A | 1-21 |
| Y | JP 2009-526075 A (Merck & Co., Inc.), 16 July 2009 (16.07.2009), paragraph [0003]<br>& WO 2007/092642 A2<br>page 1, lines 20 to 22<br>& US 2010/0227903 A1 & EP 1983966 A1<br>& CA 2641451 A & KR 10-2008-0108420 A<br>& CN 101378731 A & RU 2008136198 A | 1-21 |
| Y | JP 2006-513186 A (Pfizer Products Inc.), 20 April 2006 (20.04.2006), paragraphs [0005], [0008]<br>& WO 2004/056358 A1<br>page 1, lines 7 to 10; page 2, lines 16 to 19<br>& US 2004/0185102 A1 & EP 1961419 A1<br>& CA 2508840 A & BR 317521 A<br>& AU 2003283769 A | 1-21 |
| X | Manual of Therapeutic Agents 2007, Igaku-Shoin Ltd., 01 February 2007 (01.02.2007), pages 544, 545 | 23-29 |
| Y | BYRN S, et al., Pharmaceutical solids: a strategic approach to regulatory considerations, Pharm Res., 1995.07, Vol.12, No.7, p945-954 (p945 left column, p946 right column, p952 right column) | 9-11,18-20, 27-29 |
| Y | BAVIN M, Polymorphism in process development. (crystal structure transformations), Chemistry and Industry, 1989.08.21, (16), p527-529 (p527 left column, p528 left column) | 9-11,18-20, 27-29 |

Form PCT/ISA/210 (continuation of second sheet) (January 2015)

**INTERNATIONAL SEARCH REPORT**

International application No.
PCT/JP2015/083452

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 2014/192903 A1 (Kowa Co., Ltd.), 04 December 2014 (04.12.2014), claims & JP 2015-7038 A & TW 201512175 A | 27-29 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000017164 A
- WO 2006014357 A
- WO 2004110406 A
- WO 2008129951 A
- WO 2011152508 A
- WO 2012046681 A
- US 4231938 B
- JP 57002240 A
- US 4346227 B
- US 4444784 B
- JP SHO60500015 W
- US 5354772 B
- JP HEI358967 A
- US 5273995 B
- JP HEI1279866 A
- US 5856336 B
- JP 5178841 A
- US 5260440 B
- JP 2014064372 W


### Non-patent literature cited in the description


- *Folia Pharmacol. Jpn.,* 2007, vol. 129, 267-270
- **KUHNAST S. et al.** *Eur. Heart J.,* 20 August 2014
- *Circulation,* 2008, vol. 117, 2515-2522
- **USUI et al.** *Clin. Chem.,* 2000, vol. 46, 63-72